(19) Europäisches Patentamt
European Patent Office
Office européen des brevets

(11) EP 3 511 944 A1

(12) EUROPEAN PATENT APPLICATION

(43) Date of publication:
17.07.2019 Bulletin 2019/29

(51) Int Cl.:
G16H 50/30 (2018.01)    G16H 50/20 (2018.01)

(21) Application number: 18290007.6

(22) Date of filing: 11.01.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(71) Applicant: Ad Scientiam
75007 Paris (FR)

(72) Inventor: Cattenoz, Mathieu
92240 Malakoff (FR)

(74) Representative: Betten & Resch
Patent- und Rechtsanwälte PartGmbB
Maximiliansplatz 14
80333 München (DE)

(54) TOUCHSCREEN-BASED HAND DEXTERITY TEST

(57) Methods for a touchscreen-based hand dexterity test are provided. In one aspect, a method includes providing for display, on a touchscreen, a model path. The method also includes receiving, from the touchscreen, a plurality of user touch points. The method also includes fitting the plurality of user touch points to a user path. The method also includes sampling an array of user points along the user path and sampling an array of model points along the model path. The method also includes associating each model point in the array of model points with a corresponding user point in the array of user points, wherein the associating enforces a perpendicularity constraint. The method also includes determining a test result based on the associating. Systems and machine-readable media are also provided.

FIG. 4A

**Description**

TECHNICAL FIELD

The present disclosure generally relates to healthcare assessment tools for assessing disease progression, and more specifically relates to a touchscreen-based hand dexterity test.

BACKGROUND

Healthcare assessment tools allow a healthcare provider to track the progress of a patient's condition, such as a progression of a disease. The patient's progress can be reported to a healthcare professional for further interpretation and analysis. Patients may be encouraged to take advantage of electronic health ("E-health") initiatives, for example, by tracking their own progress using tests at home. By using electronic devices readily available to the patient, such as a smartphone or tablet, patients can perform checkups at their own convenience, rather than having to schedule frequent in-person checkups. However, present E-health approaches for healthcare assessment may not provide sufficient accuracy to enable the patient and care provider to make the most informed decisions.

The description provided in the background section should not be assumed to be prior art merely because it is mentioned in or associated with the background section. The background section may include information that describes one or more aspects of the subject technology.

SUMMARY

The disclosed system provides for a touchscreen-based hand dexterity test for healthcare assessment. A model path is shown on a touchscreen, and the user is prompted to trace a path that follows the model path as closely as possible. The paths are compared segment-by-segment to measure distances or deviations between the two paths, with constraints to select the most appropriate segments for comparison. Based on the measured distances, a test result can be provided to measure the user's dexterity or fine motor skills. A healthcare provider may report the test result to a healthcare professional, who may analyze the result to track and assess conditions affecting fine motor control, such as multiple sclerosis.

According to certain aspects of the present disclosure, a system is provided. The system includes a means for displaying a model path and for receiving a plurality of user touch points. The system includes a means for sampling an array of user points along the user path, for sampling an array of model points along the model path, for associating model points in the array of model points with corresponding user points in the array of user points, wherein a next model point is associated with a next user point based on a present user point being at least a user interval distance from the next user point on the user path, the user interval distance includes a model interval distance from a present model point to the next model point on the model path, and the user interval distance includes a correction component based on an interval error that is a scalar product of a first vector, from the present model point to the present user point, and a second vector, from the present model point to the next model point, and for determining a test result based on the associating.

According to certain aspects of the present disclosure, a computer-implemented method is provided. The method includes providing for display, on a touchscreen, a model path. The method also includes receiving, from the touchscreen, a plurality of user touch points. The method also includes fitting the plurality of user touch points to a user path. The method also includes sampling an array of user points along the user path and sampling an array of model points along the model path. The method also includes associating model points in the array of model points with corresponding user points in the array of user points, wherein a next model point is associated with a next user point based on a present user point being at least a user interval distance from the next user point on the user path, the user interval distance includes a model interval distance from a present model point to the next model point on the model path, and the user interval distance includes a correction component based on an interval error that is a scalar product of a first vector, from the present model point to the present user point, and a second vector, from the present model point to the next model point. The method also includes determining a test result based on the associating.

According to certain aspects of the present disclosure, a system is provided including a touchscreen, a memory including a model path, and a processor configured to execute instructions. When executed, the instructions cause the processor to provide for display, on the touchscreen, the model path. The instructions also cause the processor to receive, from the touchscreen, a plurality of user touch points. The instructions also cause the processor to fit the plurality of user touch points to a user path. The instructions also cause the processor to sample an array of user points along the user path and sample an array of model points along the model path. The instructions also cause the processor to associate each model point in the array of model points with a corresponding user point in the array of user points, wherein a next model point is associated with a next user point based on a present user point being at least a user interval distance from the next user point on the user path, the user interval distance includes a model interval distance from a present model point to the next model point on the model path, and the user interval distance includes a correction component based on an interval error that is a scalar product of a first vector, from the present model point to the present user point, and a second vector, from the present model point to the next model point. The instructions also cause the processor to determine a test result based on the associating.

According to certain aspects of the present disclosure, a non-transitory machine-readable storage medium is provided that includes machine-readable instructions for causing a processor to execute a method. The method includes providing for display, on a touchscreen, a model path. The method includes receiving, from the touchscreen, a plurality of user touch points. The method includes fitting the plurality of user touch points to a user path. The method includes sampling an array of user points along the user path and sampling an array of model points along the model path. The method includes associating each model point in the array of model points with a corresponding user point in the array of user points, wherein a next model point is associated with a next user point based on a present user point being at least a user interval distance from the next user point on the user path, the user interval distance includes a model interval distance from a present model point to the next model point on the model path, and the user interval distance includes a correction component based on an interval error that is a scalar product of a first vector, from the present model point to the present user point, and a second vector, from the present model point to the next model point, wherein the correction component is applied over a plurality of model points. The method includes determining a test result based on the associating.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

BRIEF DESCRIPTION OF THE DRAWINGS

The accompanying drawings, which are included to provide further understanding and are incorporated in and constitute a part of this specification, illustrate aspects of the subject technology, and together with the description serve to explain the principles of the subject technology. In the drawings:

FIG. 1 illustrates an example architecture for a touchscreen-based hand dexterity test.

FIG. 2 is a block diagram illustrating the example client from the architecture of FIG. 1 according to certain aspects of the disclosure.

FIG. 3A illustrates an example process for a touchscreen-based hand dexterity test using the example client of FIG. 2.

FIG. 3B illustrates an example process for iteratively associating each model point in an array of model points with a corresponding user point in an array of user points using the example client of FIG. 2.

FIG. 4A and FIG. 4B illustrate example user interfaces for practicing the example process of FIG. 3A.

FIG. 5A illustrates an example user path and model path for a touchscreen-based hand dexterity test.

FIG. 5B illustrates distances determined between the example paths of FIG. 5A without matching constraints.

FIG. 5C illustrates distances determined between the example paths of FIG. 5A with matching constraints.

FIG. 6 illustrates example paths for practicing the example process of FIG. 3B.

FIG. 7 is a block diagram illustrating an example computer system with which the client of FIG. 2 can be implemented.

In one or more implementations, not all of the depicted components in each figure may be required, and one or more implementations may include additional components not shown in a figure. Variations in the arrangement and type of the components may be made without departing from the scope of the subject disclosure. Additional components, different components, or fewer components may be utilized within the scope of the subject disclosure.

DETAILED DESCRIPTION

The detailed description set forth below is intended as a description of various implementations and is not intended to represent the only implementations in which the subject technology may be practiced. As those skilled in the art would realize, the described implementations may be modified in various different ways, all without departing from the scope of the present disclosure. Accordingly, the drawings and description are to be regarded as illustrative in nature and not restrictive.

General Overview

The disclosed system provides for a touchscreen-based hand dexterity test for healthcare assessment. A model path is shown on a touchscreen, and the user is prompted to trace a path that follows the model path as closely as possible. The paths are associated segment-by-segment to measure distances or deviations between the two paths, with constraints to select the most appropriate segments for association. Based on the measured distances, a test result can be provided to measure the user's dexterity or fine motor skills. A healthcare provider may provide the test result to a healthcare professional to track and assess conditions affecting fine motor control, such as multiple sclerosis.

The disclosed system addresses a technical problem tied to computer technology and arising in the realm of computer networks, namely the technical problem of touchscreen-based dexterity tests providing insufficient accuracy. A healthcare provider may request a user to perform a touchscreen-based dexterity test and transmit the test result to the healthcare provider over a network. The touchscreen-based dexterity test may compare a received user path to a model path without any matching constraints. For model paths with more complex shapes such as a spiral shape, false matches may be made to segments on the user path with respect to the model path. Further, due to human behavior such as unintentionally overshooting or undershooting a shape or curve, drifting away from the model path, taking path shortcuts, or adding path noise due to hand tremors, vibrations, or other factors, the user path may deviate more than expected from the model path if the human behavior is not taken into consideration. As a result, the user's hand dexterity may be underestimated or overestimated.

The disclosed system solves this technical problem by enforcing matching constraints when comparing the user path to the model path, thereby providing improved measurement accuracy. Additional features are also disclosed, such as curve fitting the user path, enforcing constant interval distances between sampled points of a model path, and gradually adjusting matching constraints by computing and applying a correction component over multiple points. The disclosed system thus improves the field of computer assisted healthcare assessment by providing a more accurate touchscreen-based dexterity test.

Although certain examples provided herein may describe a user's data and/or test results being stored in memory, each user must grant explicit permission for such user information to be stored. The explicit permission may be granted using privacy controls integrated into the disclosed system. If requested user information includes demographic information, then the demographic information is aggregated on a group basis and not by individual user. Each user is provided notice that such user information will be stored with such explicit consent, and each user may at any time end having the user information stored, and may delete the stored user information. The stored user information may be encrypted to protect user security.

The user can at any time delete the user information from memory and/or opt out of having the user information stored in memory. Additionally, the user can, at any time, adjust appropriate privacy settings to selectively limit the types of user information stored in memory, or select the memory in which the user information is stored (e.g., locally on the user's device as opposed to remotely on a server). For example, the user may specify that the user information is only stored locally on the user's device and not to be transmitted externally. In many examples, the user information does not include and/or share the specific identification of the user (e.g., the user's name) unless otherwise specifically provided or directed by the user.

Example System Architecture

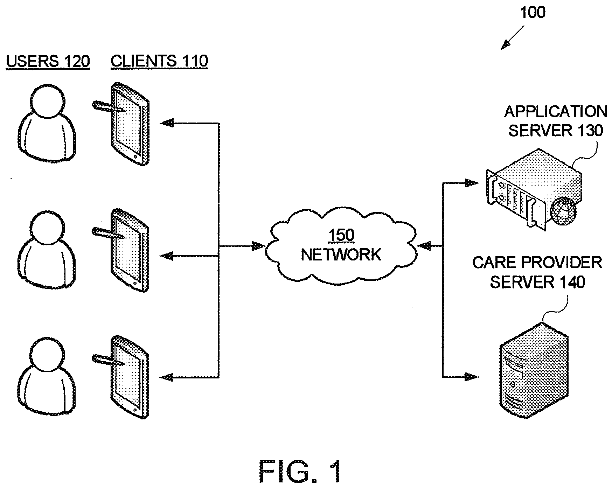

FIG. 1 illustrates an example architecture 100 for a touchscreen-based hand dexterity test. The architecture 100 includes clients 110, application server 130, and care provider server 140 connected over a network 150. Users 120 may further interact with respective clients 110.

The clients 110 may provide a touchscreen-based hand dexterity test for respective users 120. For example, clients 110 may have previously downloaded an E-health tracking application from application server 130 over network 150, and the E-health tracking application may provide the dexterity test as a selectable test. After users 120 perform the dexterity test, clients 110 may send data and/or test results to care provider server 140. Care provider server 140 may associate the received data with patient profiles for users 120, and may further perform analysis on the patient profiles, both individually and aggregately, to provide healthcare professionals with recommendations and/or adjustments for the treatment plans of users 120.

The clients 110 can be any device having an appropriate processor, memory, and communications capability for retrieving and executing the E-health tracking application. The clients 110 to which the servers 130 are connected over the network 150 can be, for example, desktop computers, mobile computers, tablet computers (e.g., including e-book readers), mobile devices (e.g., a smartphone or PDA), set top boxes (e.g., for a television), video game consoles, or any other devices having appropriate processor, memory, and communications capabilities. Clients 110 may include touchscreens, pen digitizers, or other devices for receiving input from the hands of users 120. In certain aspects, one or more of the servers 130 can be a cloud computing server of an infrastructure-as-a-service (IaaS), and be able to support a platform-as-a-service (PaaS) and software-as-a-service (SaaS) services.

The network 150 can include, for example, any one or more of a personal area network (PAN), a local area network (LAN), a campus area network (CAN), a metropolitan area network (MAN), a wide area network (WAN), a broadband network (BBN), the Internet, and the like. Further, the network 150 can include, but is not limited to, any one or more of the following network topologies, including a bus network, a star network, a ring network, a mesh network, a star-bus network, tree or hierarchical network, and the like.

Example Touchscreen-based Hand Dexterity Testing System

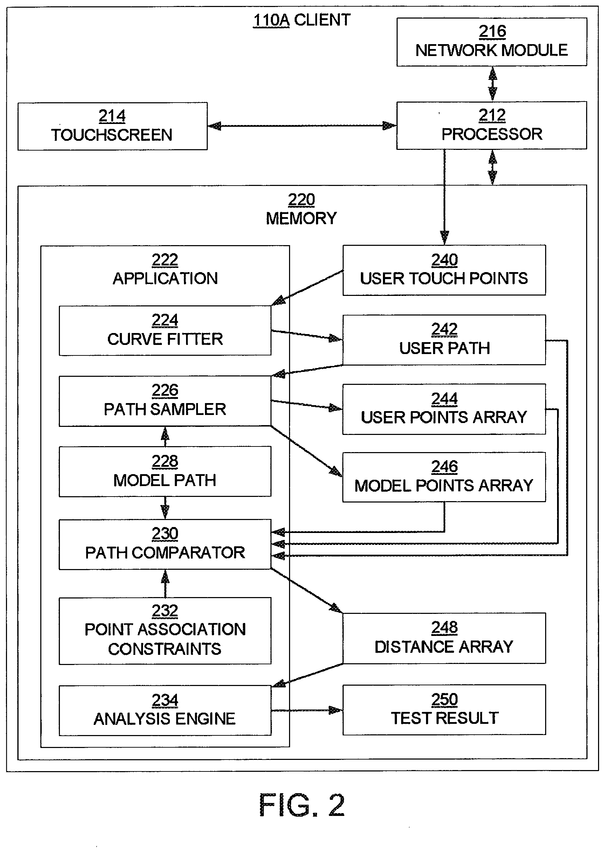

FIG. 2 is a block diagram illustrating an example client 110A from the architecture of FIG. 1 according to certain aspects of the disclosure. Client 110A includes processor 212, touchscreen 214, network module 216, and memory 220 that includes application 222, user touch points 240, user path 242, user points array 244, model points array 246, distance array 248, and test result 250. Application 222 includes curve fitter 224, path sampler 226, model path 228, path comparator 230, point association constraints 232, and analysis engine 234.

The processor 212 of the client 110A is configured to execute instructions, such as instructions physically coded into the processor 212, instructions received from software in memory 220, or a combination of both. For example, the processor 212 of the client 110A executes application 222 to provide for display, on touchscreen 214, a model path 228. The model path 228 corresponds to a path or shape that the user is prompted to trace with a finger or a handheld input device. For example, model path 228 may correspond to a straight line, zigzag, circle, wave, or spiral shape, and may be expressed as a composite Bezier curve.

Processor 212 executes application 222 to receive, from touchscreen 214, user touch points 240. For example, the user may use one or more fingers to follow model path 228, and application 222 may sample a number of user touch points 240 from touchscreen 214 at a sampling or polling rate supported by touchscreen 214, for example 60 times per second. Each of user touch points 240 may include a coordinate or $(x, y)$ position corresponding to the location of the sampled touch on touchscreen 214. Optionally, user touch points 240 may also include pressure values and/or tilt values when supported by touchscreen 214.

Processor 212 executes curve fitter 224 to fit user touch points 240 to user path 242. For example, when user path 242 is to be stored as a composite Bezier curve, algorithms for working with Bezier curves can be utilized to organize user touch points 240 into a continuous composite curve of individually connected Bezier curve segments that are best-fit matched to corresponding points within user touch points 240.

Processor 212 executes path sampler 226 to sample user points array 244 along user path 242 and sample model points array 246 along model path 228. Since a curve for model path 228 is defined in memory 220 and a curve for user path 242 is defined after executing curve fitter 224, path sampler 226 may trace each defined curve and sample points or $(x, y)$ coordinates at fixed intervals to obtain a desired number of samples. Note that a number of samples for model path 228 and user path 242 may be set independently, with a larger number of samples providing higher accuracy with a higher computational cost.

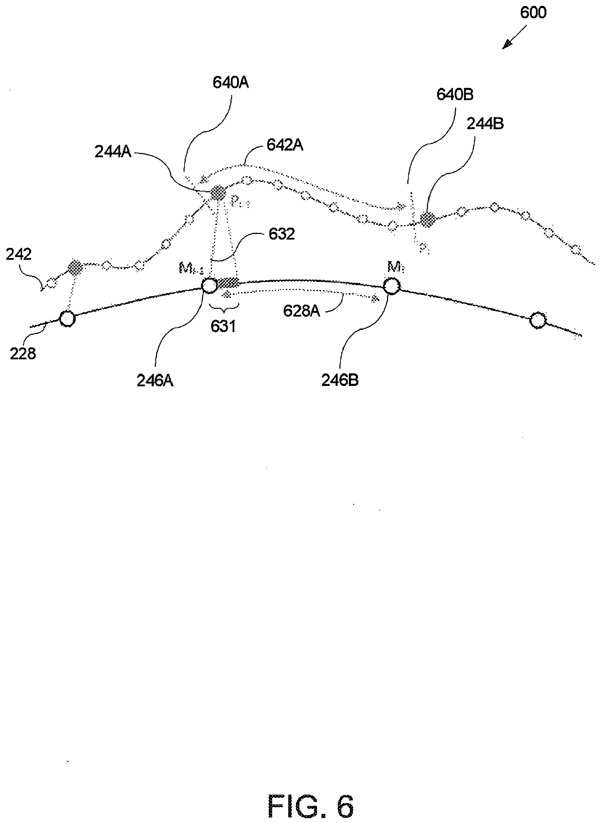

Processor 212 executes path comparator 230 to associate each model point in model points array 246 with a corresponding user point in user points array 244. Path comparator 230 enables a distance or deviation to be estimated between user path 242 and model path 228 by comparing selected points from the arrays output by path sampler 226, or user points array 244 and sample model points array 246. To select points for comparison, path comparator 230 enforces point association constraints 232, as discussed in further detail below in conjunction with FIG. 6. Distance array 248 can be determined based on the associating of path comparator 230. For example, each distance vector from a point in model points array 246 to a corresponding point in user points array 244 may be stored in distance array 248.

Processor 212 can execute analysis engine 234 to determine a test result 250 based on distance array 248. For example, analysis engine 234 may calculate an average distance between user path 242 and model path 228 by summing magnitudes in distance array 248 and dividing by a size of distance array 248. This average distance may then be compared to threshold ranges to provide a test result 250 that scores the user's dexterity. For example, an average distance from 0 cm to 3 cm might be considered an excellent score, an average distance greater than 3 cm but less than 6 cm might be considered a normal score, and an average distance of 6 cm or higher might be considered a marginal score that merits further review by a healthcare professional.

Processor 212 can execute application 222 to send test result 250 to care provider server 140 via network module 216 over network 150. In alternative implementations, analysis engine 234 can be located remotely, such as on care provider server 140, in which case processor 212 can execute application 222 to send distance array 248 to care provider server 140 via network module 216 over network 150. The processor 212 of client 110A executes instructions from application 222 causing the processor 212 to provide a touchscreen-based hand dexterity test.

In one or more aspects of the subject technology, client 110A provides a touchscreen-based hand dexterity test, the client 110A including a touchscreen 214, a memory 220 including a model path 228, and a processor 212 configured to execute application 222 which, when executed, causes processor 212 to provide for display, on touchscreen 214, model path 228, receive, from touchscreen 214, a user touch points 240, fit user touch points 240 to user path 242, sample user points array 244 along user path 242 and sample model points array 246 along model path 228, associate each model point in model points array 246 with a corresponding user point in user points array 244, wherein a next model point is associated with a next user point based on a present user point being at least a user interval distance from the next user point on user path 242, the user interval distance includes a model interval distance from a present model point to the next model point on model path 228 and the user interval distance includes a correction component based on an interval error that is a scalar product of a first vector, from the present model point to the present user point, and a second vector, from the present model point to the next model point, and determine a test result 250 based on the associating.

In a further aspect of the subject technology, processor 212 is configured to determine test result 250 by storing, in memory 220, a distance array 248 including a magnitude of the first vector from each model point of the array of model points, and analyzing distance array 248 to determine test result 250. For example, as discussed above, averaging and comparing to threshold ranges may be one approach to determine test result 250.

In a further aspect of the subject technology, processor 212 is configured to analyze distance array 248 by using a global distance including a sum of distance array 248 to determine test result 250. For example, as discussed above, the distances of distance array 248 may be summed and divided by the size of distance array 248 to determine an average distance, and the average distance may be compared to threshold ranges to determine test result 250.

In a further aspect of the subject technology, model path 228 includes a spiral shape. Other shapes such as a straight line, zigzag, circle, or wave can also be included.

In a further aspect of the subject technology, processor 212 is configured to apply the correction component over a plurality of model points. By applying the correction component over a larger number of samples, sudden changes in the user interval distance are avoided, which may help to provide a more accurate result and compensate for potential noise in user path 242.

In a further aspect of the subject technology, adjacent points in model points array 246 are separated by a constant interval distance. By using a constant interval distance, samples are distributed evenly throughout entire model path 228, which allows distance deviations to have the same influence or weight regardless of the relative position within model path 228.

In a further aspect of the subject technology, model path 228 and user path 242 each include composite Bezier curves. Bezier curves may be particularly suited for computer based rendering and manipulation, with a composite curve allowing more complex shapes to be represented as connected segments of simpler curves.

The techniques described herein may be implemented as method(s) that are performed by physical computing device(s), as one or more non-transitory computer-readable storage media storing instructions which, when executed by computing device(s), cause performance of the method(s), or, as physical computing device(s) that are specially configured with a combination of hardware and software that causes performance of the method(s).

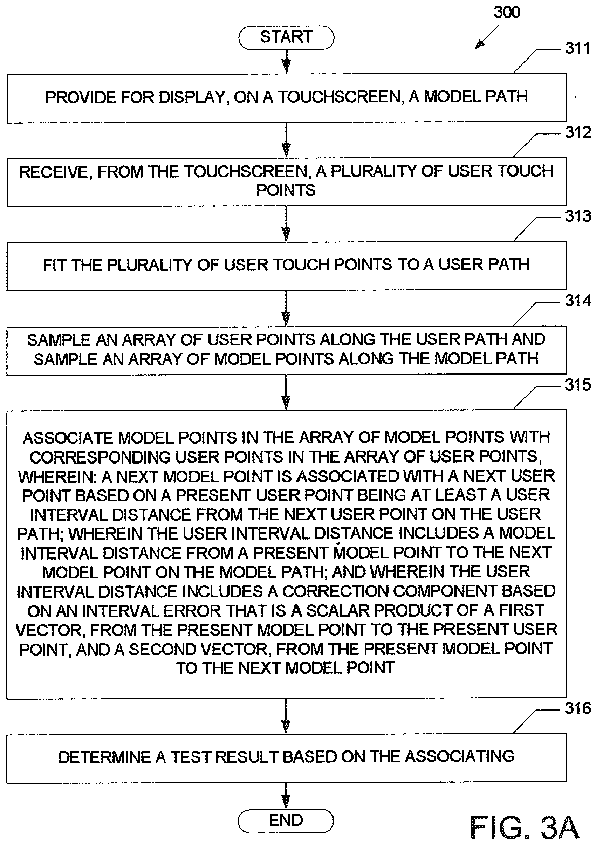

FIG. 3A illustrates an example process 300 for providing a touchscreen-based hand dexterity test using the example client 110A of FIG. 2. While FIG. 3A is described with reference to FIG. 2, it should be noted that the process steps of FIG. 3A may be performed by other systems.

Prior to process 300, application 222 may have been previously downloaded from application server 130 and installed onto client 110A. Based on a preset testing schedule or in response to an instruction sent from care provider server 140, an operating system of client 110A may, for example, issue a push notification for application 222 notifying that a test is due for the user. The user may interact with the push notification to launch a corresponding test of application 222, such as a hand dexterity test, to initiate process 300.

The process 300 begins by proceeding to step 311, where processor 212 provides for display, on touchscreen 214, model path 228. Model path 228 can be any shape such as a straight line, zigzag, circle, wave, or spiral. In some aspects of the subject technology, model path 228 may be selected from a shape database stored in application 222, or computed by a specific program, or accessible via network 150. Model path 228 may be stored as a composite Bezier curve, allowing application 222 to easily scale model path 228 according to a resolution of touchscreen 214 and to sample points along model path 228.

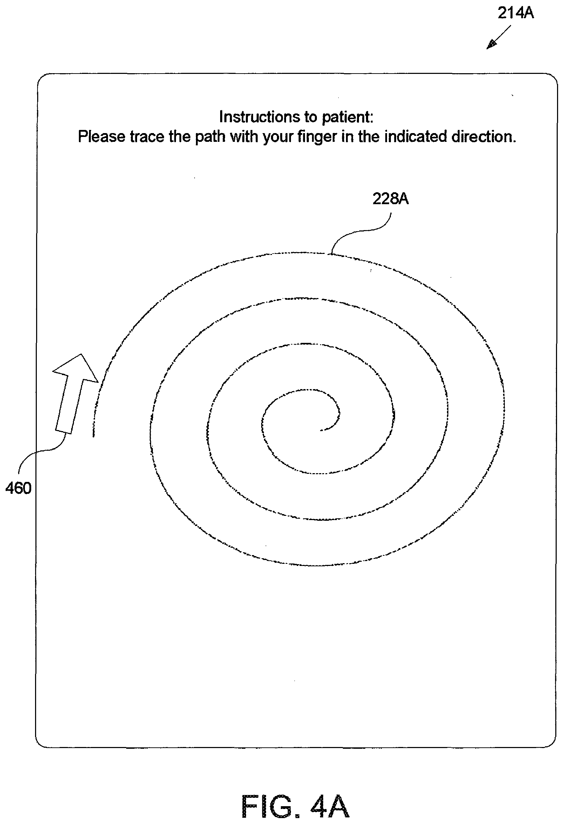

For example, FIG. 4A illustrates an example user interface for practicing step 311. Touchscreen 214A of FIG. 4A includes model path 228A as a spiral and an indicator 460 placed at an outer end of model path 228A. As shown in touchscreen 214A, a user interface is displayed that instructs the user on how to perform the hand dexterity test, namely by following model path 228A with a finger or other handheld input device along a direction indicated by indicator 460. In alternative aspects of the subject technology, indicator 460 may also be placed at an inner end of model path 228A. In some aspects of the subject technology, model path 228A may be scaled to display to a particular size on touchscreen 214A, for example by using a known resolution and pixel density (dots per inch, or DPI) of touchscreen 214A

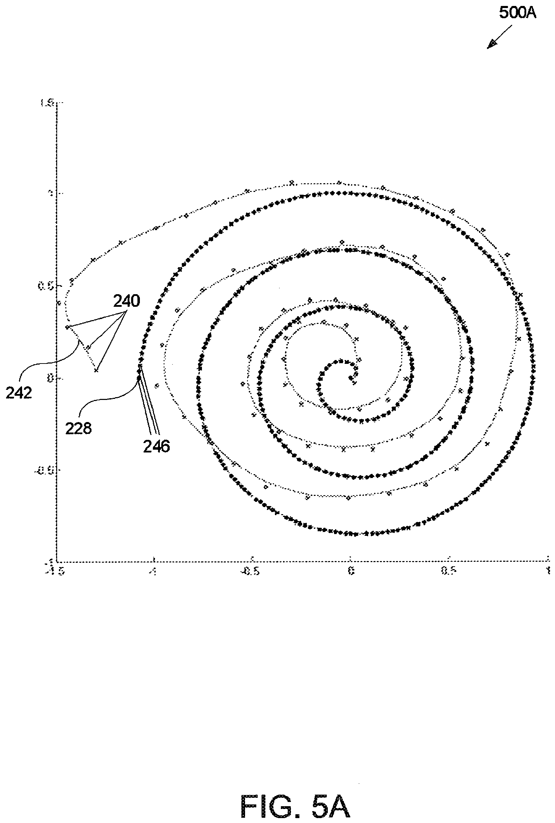

In step 312, processor 212 receives, from touchscreen 214, user touch points 240. Touchscreen 214 may sample user input at a fixed sampling rate. Thus, even if the user is continuously in contact with touchscreen 214, the inputs that are read may correspond to separate points, in accordance with the fixed sampling rate. For example, FIG. 5A illustrates an example user path 242 and model path 228 for a touchscreen-based hand dexterity test. As shown by user touch points 240, of which three are indicated in FIG. 5A, the user touch points 240 may be read as a series of points.

While the described example receives touch inputs, alternative aspects of the subject technology may use a capacitive digitizer, resistive digitizer, or another input device to support a pen, stylus, or other hand operated non-touch peripheral. Processor 212 may read the user input from the input device, and store the user input as user touch points 240 in memory 220.

In step 313, processor 212 executes curve fitter 224 to fit user touch points 240 to user path 242. For example, curve fitter 224 may use a curve fitting algorithm to generate user path 242 from user touch points 240. The curve fitting algorithm may use a smoothing function for best fit rather than an interpolation for exact fit, as illustrated by user path 242 in FIG. 5A. User path 242 may be stored as a composite Bezier curve, or a continuous curve composed of multiple polynomial curve segments.

In step 314, processor 212 executes path sampler 226 to sample user points array 244 along user path 242 and sample model points array 246 along model path 228. For example, referring to FIG. 5A, a first three sample points of model points array 246 are identified along model path 228. The sampled points may be stored as two dimensional X, Y coordinates. A similar sampling may be carried out for user points array 244 along user path 242. Note that both user path 242 and model path 228 may be sampled along the same direction, or from outside the spiral travelling inwards. Further, note that a number of samples provided by user points array 244 may be greater than a number of samples provided by user touch points 240, thereby providing higher granularity compared to using user points array 244 directly.

A number of samples for user points array 244 and model points array 246 may be set based on balancing accuracy against available computational resources, such as processor cycles, free memory, and network bandwidth. A greater number of samples may allow for a more accurate test at the cost of higher computational complexity, memory footprint, and network utilization.

To provide an even measurement attribution along model path 228, model points array 246 may be sampled from model path 228 such that adjacent samples are separated by a constant interval distance. For example, if model path 228 in FIG. 5A were to be sampled from model path 228 based on constant angle intervals of a parametric equation, then a larger number of samples would be clustered towards the center of the spiral, causing a measurement bias near the center of the spiral. By enforcing a constant interval distance within model points array 246, measurements may be weighted equally regardless of position within model path 228.

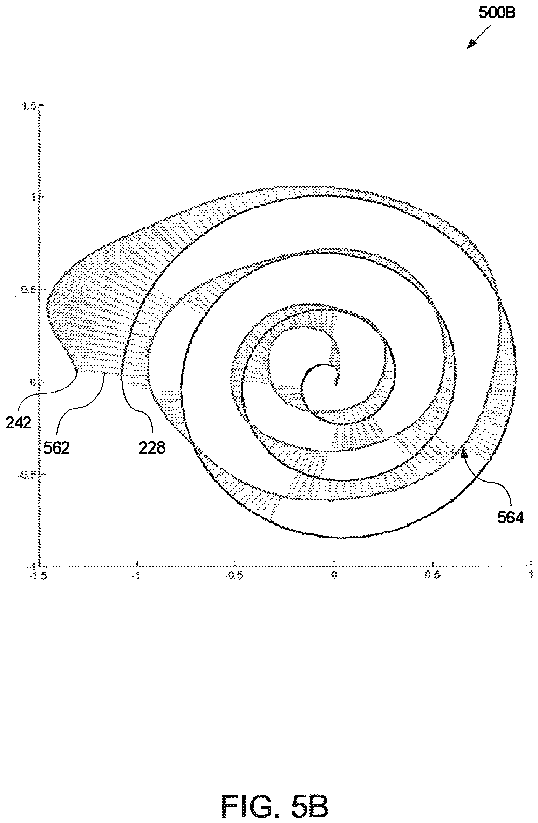

In step 315, processor 212 executes path comparator 230 to associate model points in model points array 246 with corresponding user points in user points array 244. Before describing step 315 in detail, it may be useful to consider the problems with a closest point matching algorithm along a user path, as illustrated in FIG. 5B. In FIG. 5B, each point along user path 242 is associated to a corresponding point in model path 228 according to minimizing a distance 562 between the two points. While satisfactory results may be provided when model path 228 is a simple shape, the algorithm begins to exhibit problems as model path 228 becomes more complex, such as a spiral shape, or as user path 242 becomes more erratic. For example, as shown in FIG. 5B, the point associating becomes discontinuous at point 564 and erroneously matches a user point to an inner portion of model path 228, rather than to an outer portion of model path 228.

Thus, in step 315, path comparator 230 may enforce point association constraints 232 when performing the point associating. Consecutively matched points in user points array 244 may be separated by a user interval distance along user path 242 that is based on a model interval distance between corresponding points in model points array 246. Consecutively matched points in model points array 246 may also be separated by a specific interval distance along model path 228. Since the user is following model path 228 with a finger, user path 242 can be expected to approximately follow model path 228, and therefore the user interval distance can be expected to be approximately the same as the model interval distance.

Since the user cannot trace model path 228 exactly, portions of user path 242 may undershoot (smaller curve) or overshoot (larger curve) in comparison to model path 228. Accordingly, point association constraints 232 may also consider a deformation of user path 242 with respect to model path 228 by adjusting the user interval distance by a correction component. This correction component is based on an interval error that is the scalar product of two vectors from a model point: a first vector extending to an associated user point, and a second vector extending to the next model point, wherein the second vector may be a normalized vector.

When a model point being considered is already associated with a known user point, then an expected position of the next user point to be matched can be determined by moving a reference position along the user path by the user interval distance adjusted by the correction component. The next point in user points array 244 after the reference position in user path 242 will be associated with the next point in model points array 246, and the matching can continue to iterate for the remaining points along model points array 246.

Note that while the example above associates points from model points array 246 to points on user points array 244, the association may also proceed in the opposite direction. In other words, points on user points array 244 may be iterated and associated with points on model points array 246. Further, in some aspects of the present technology, every other or every Nth point may be iterated in model points array 246 or user points array 244. Thus, some points may not be associated to another point at all, and some points may be associated to multiple corresponding points.

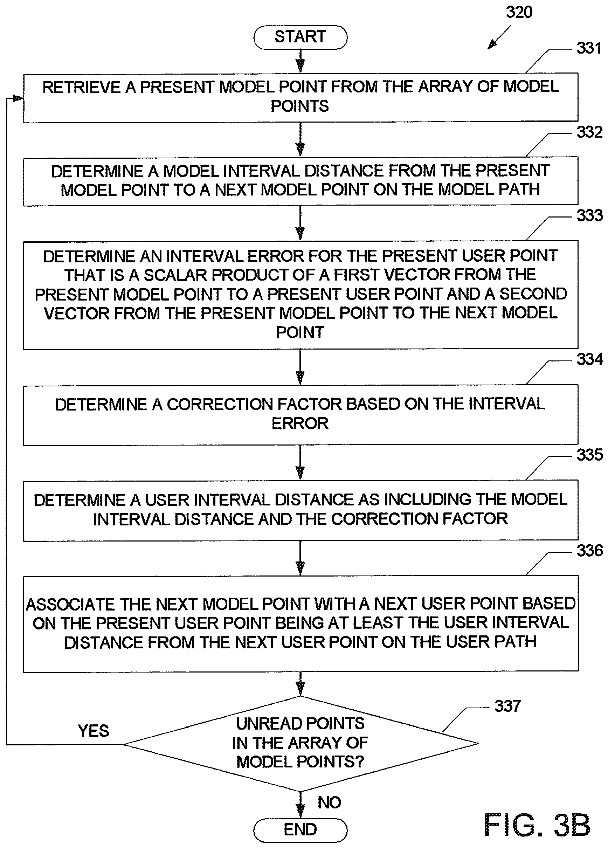

A more detailed description of the processing in step 315 is discussed below in conjunction with FIG. 3B and FIG. 6, which illustrates example paths for practicing the example process of FIG. 3B. Diagram 600 of FIG. 6 includes user path 242, model path 228, present user point 244A, next user point 244B, present model point 246A, next model point 246B, model interval distance 628A, interval error 631, vector 632, reference position 640A, reference position 640B, and user interval distance 642A.

FIG. 3B illustrates an example process 320 for iteratively associating each model point in model points array 246 with a corresponding user point in user points array 244 using the example client 110A of FIG. 2. While FIG. 3B is described with reference to FIG. 2 and FIG. 6, it should be noted that the process steps of FIG. 3B may be performed by other systems.

The process 320 begins by proceeding to step 331, where processor 212 retrieves a present model point 246A from model points array 246. Step 331 assumes that present model point 246A is already associated with a corresponding present user point 244A, for example from a previous iteration of process 320. If process 320 begins without any prior iterations, then an initial association may be made, for example by examining the first several user points in user points array 244 and assigning a particular user point to the first model point of model points array 246 based on minimizing distance. Of course, this is only one example for an initial association, and other methods may be utilized.

In step 332, processor 212 determines model interval distance 628A from present model point 246A to next model point 246B on model path 228. For example, when model path 228 is a composite Bezier curve, then model interval distance 628A can be calculated using distance calculations for Bezier curves. When a constant interval distance is maintained between adjacent points in model points array 246, then the calculations in step 332 may be skipped and model interval distance 628A can be preset to the constant interval distance.

In step 333, processor 212 determines interval error 631 for present model point 246A that is a scalar product of a first vector from present model point 246A to present user point 244A and a second vector from present model point 246A to next model point 246B. In FIG. 6, the first vector is from $M_{i-1}$ to $P_{i-1}$, and the second vector is from $M_{i-1}$ to $M_i$. The second vector may be normalized. The result of the scalar product is interval error 631.

In step 334, processor 212 determines a correction component based on interval error 631. For example, a proposed correction component is provided below:

$$\textit{Equation 1:} \quad \text{interval\_correction} = (\text{-interval\_error}) * 1/N + \text{interval\_correction} * (1\text{-}1/N)$$

wherein interval correction is the correction component, interval error is interval error 631, and N is a number of points to average the correction component. By setting N > 1, the correction component is averaged over a plurality of points, effectively smoothing the correction so that distances do not change suddenly. This averaging may help to make the measurement less sensitive to potential noise on user path 242.

In step 335, processor 212 determines user interval distance 642A as including model interval distance 628A and the correction component of step 334. For example:

$$\textit{Equation 2:} \quad \text{path\_user\_interval\_min} = \text{path\_model\_interval} + \text{interval\_correction}$$

wherein path_user_interval min is user interval distance 642A, path model interval is model interval distance 628A, and interval_correction is the correction component from step 334. Thus, referring to FIG. 6, user interval distance 642A is a distance that at least includes model interval distance 628A and the correction component of step 334.

It should be noted that the decision to constrain user interval distance 642A to contain at least model interval distance 628A is only one approach for approximating user interval distance 642A. Another approach may constrain the user interval distance 642A to not exceed model interval distance 628A, and yet another approach may constrain the user interval distance 642A to be as close as possible to a next user interval distance starting from next user point 244B. However, for purposes of computational simplicity, the approach of constraining user interval distance 642A to contain at least model interval distance 628A has been adopted for step 335.

In step 336, processor 212 associates next model point 246B with next user point 244B based on present user point 244A being at least user interval distance 642A from next user point 244B on user path 242. Since user points array 244 is an array of discrete points rather than a continuous user path 242, a separate reference position 640A may be helpful to maintain accuracy while following user path 242. Thus, reference position 640A may correspond to a projected matching point for present model point 246A, for which present user point 244A is the next available point in user points array 244. To determine the next user point to match to next model point 246B, reference position 640A may be advanced by user interval distance 642A along user path 242, resulting in reference position 640B. The next available point after reference position 640B in user points array 244 is next user point 244B, which is accordingly associated with next model point 246B. Thus, an association between a segment of model path 228 and user path 242 is completed.

In step 337, processor 212 determines if there are unread points in model points array 246. If the answer to step 337 is yes, then process 320 may return to step 331 for iteration. Otherwise, the answer to step 337 is no, and process 320 ends.

As shown in FIG. 6, when a vector 632 is added to connect a first vector to a second vector, a triangle with a right angle is formed. Thus, point association constraints 232 enforces a perpendicularity constraint when matching model points to user points, as achieved via process 320 described above.

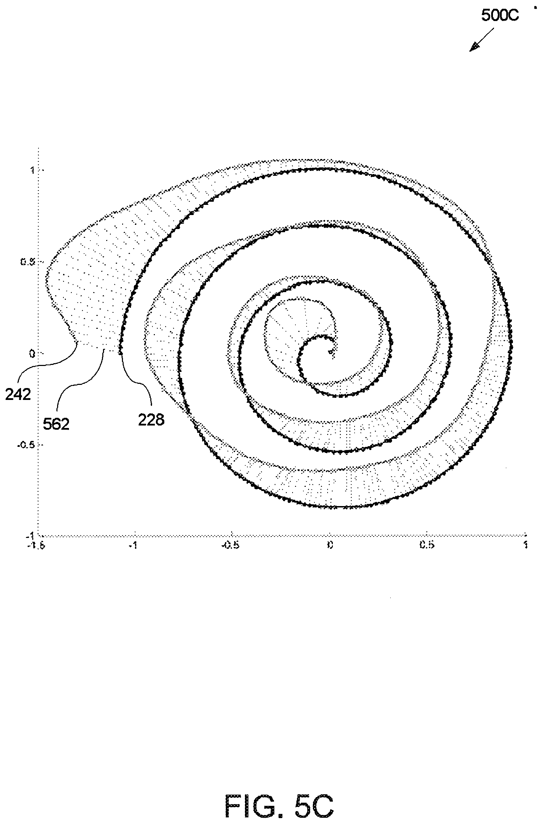

After process 320 completes, corresponding to a completion of step 315, distances between associated points may be represented as in FIG. 5C. FIG. 5C illustrates distances 562 determined between model path 228 and user path 242 with matching constraints. As shown in FIG. 5C, the matching along user path 242 is continuous, unlike FIG. 5B. Distances 562 for each model point in model points array 246 may be stored as vectors in distance array 248. In some aspects of the subject technology, distance array 248 may be stored as an array of magnitudes, thereby discarding the directional information from the vectors. Thus, distance array 248 can be flattened down to a single dimensional array, which may facilitate data analysis.

In step 316, processor 212 executes analysis engine 234 to determine a test result based on the associating in step 315. Analysis engine 234 may analyze distance array 248 to determine the test result. For example, a global distance may be computed that is a sum of single dimensional distance array 248. This global distance may be divided by the number of model points in model points array 246 to determine an average distance or deviance. This average distance may then be compared to thresholds to determine a dexterity level of the user, wherein lower thresholds indicate higher hand dexterity. Of course, this is only one example; analysis engine 234 may use any algorithm on distance array 248. Moreover, in some aspects of the subject technology, analysis engine 234 may be executed remotely, for example on care provider server 140, in which case distance array 248 may be sent to care provider server 140.

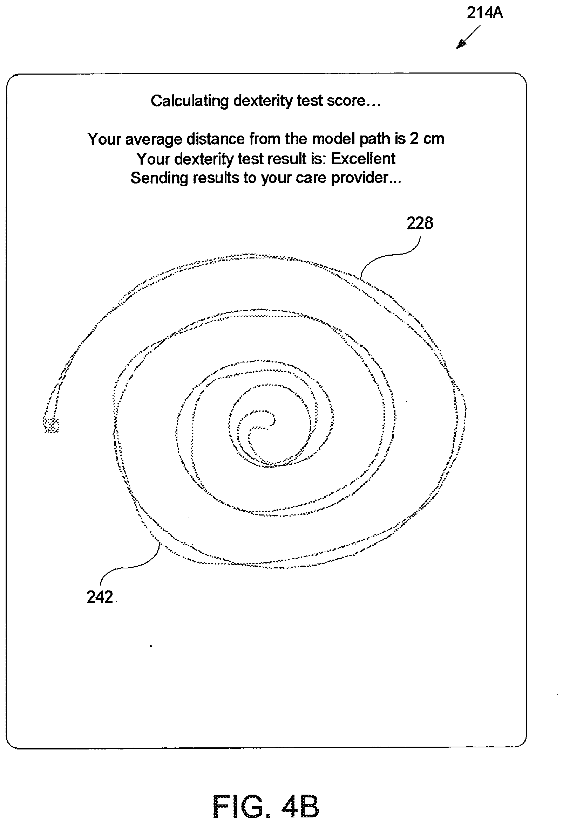

The test result may be conveyed to the user, as shown in FIG. 4B. FIG. 4B illustrates an example user interface for practicing the example process of FIG. 3A. As shown in FIG. 4B, the user is informed on touchscreen 214A that user path 242 deviates from model path 228 by an average of 2 cm, and that the test result is "Excellent." The test result may also be sent to care provider server 140, and the process 300 ends.

Hardware Overview

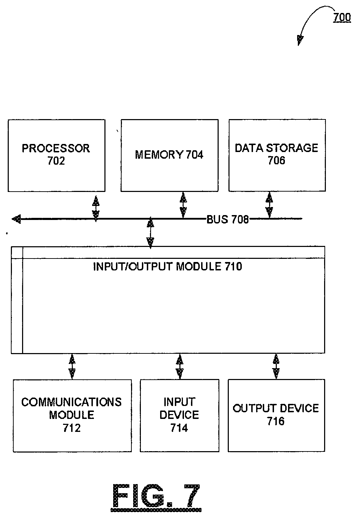

FIG. 7 is a block diagram illustrating an example computer system 700 with which the client 110A of FIG. 2, 3A and 3B can be implemented. In certain aspects, the computer system 700 may be implemented using hardware or a combination of software and hardware, either in a dedicated server, or integrated into another entity, or distributed across multiple entities.

Computer system 700 (e.g., client 110A) includes a bus 708 or other communication mechanism for communicating information, and a processor 702 (e.g., processor 212) coupled with bus 708 for processing information. According to one aspect, the computer system 700 can be a cloud computing server of an IaaS that is able to support PaaS and SaaS services. According to one aspect, the computer system 700 is implemented as one or more special-purpose computing devices. The special-purpose computing device may be hard-wired to perform the disclosed techniques, or may include digital electronic devices such as one or more application-specific integrated circuits (ASICs) or field programmable gate arrays (FPGAs) that are persistently programmed to perform the techniques, or may include one or more general purpose hardware processors programmed to perform the techniques pursuant to program instructions in firmware, memory, other storage, or a combination. Such special-purpose computing devices may also combine custom hard-wired logic, ASICs, or FPGAs with custom programming to accomplish the techniques. The special-purpose computing devices may be desktop computer systems, portable computer systems, handheld devices, networking devices, or any other device that incorporates hard-wired and/or program logic to implement the techniques. By way of example, the computer system 700 may be implemented with one or more processors 702. Processor 702 may be a general-purpose microprocessor, a microcontroller, a Digital Signal Processor (DSP), an ASIC, a FPGA, a Programmable Logic Device (PLD), a controller, a state machine, gated logic, discrete hardware components, or any other suitable entity that can perform calculations or other manipulations of information.

Computer system 700 can include, in addition to hardware, code that creates an execution environment for the computer program in question, e.g., code that constitutes processor firmware, a protocol stack, a database management system, an operating system, or a combination of one or more of them stored in an included memory 704 (e.g., memory 220), such as a Random Access Memory (RAM), a flash memory, a Read Only Memory (ROM), a Programmable Read-Only Memory (PROM), an Erasable PROM (EPROM), registers, a hard disk, a removable disk, a CD-ROM, a DVD, or any other suitable storage device, coupled to bus 708 for storing information and instructions to be executed by processor 702. The processor 702 and the memory 704 can be supplemented by, or incorporated in, special purpose logic circuitry. Expansion memory may also be provided and connected to computer system 700 through input/output module 710, which may include, for example, a SIMM (Single In Line Memory Module) card interface. Such expansion memory may provide extra storage space for computer system 700, or may also store applications or other information for computer system 700. Specifically, expansion memory may include instructions to carry out or supplement the processes described above, and may include secure information also. Thus, for example, expansion memory may be provided as a security module for computer system 700, and may be programmed with instructions that permit secure use of computer system 700. In addition, secure applications may be provided via the SIMM cards, along with additional information, such as placing identifying information on the SIMM card in a non-hackable manner.

The instructions may be stored in the memory 704 and implemented in one or more computer program products, e.g., one or more modules of computer program instructions encoded on a computer readable medium for execution by, or to control the operation of, the computer system 700, and according to any method well known to those of skill in the art, including, but not limited to, computer languages such as data-oriented languages (e.g., SQL, dBase), system languages (e.g., C, Objective-C, C++, Assembly), architectural languages (e.g., Java, .NET), and application languages (e.g., PHP, Ruby, Perl, Python). Instructions may also be implemented in computer languages such as array languages, aspect-oriented languages, assembly languages, authoring languages, command line interface languages, compiled languages, concurrent languages, curly-bracket languages, dataflow languages, data-structured languages, declarative languages, esoteric languages, extension languages, fourth-generation languages, functional languages, interactive mode languages, interpreted languages, iterative languages, list-based languages, little languages, logic-based languages, machine languages, macro languages, metaprogramming languages, multiparadigm languages, numerical analysis, non-English-based languages, object-oriented class-based languages, object-oriented prototype-based languages, off-side rule languages, procedural languages, reflective languages, rule-based languages, scripting languages, stack-based languages, synchronous languages, syntax handling languages, visual languages, wirth languages, embeddable languages, and xml-based languages. Memory 704 may also be used for storing temporary variable or other intermediate information during execution of instructions to be executed by processor 702.

A computer program as discussed herein does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program can be deployed to be executed on one computer or on multiple computers that are located at one site or distributed across multiple sites and interconnected by a communication network, such as in a cloud-computing environment. The processes and logic flows described in this specification can be performed by one or more programmable processors executing one or more computer programs to perform functions by operating on input data and generating output.

Computer system 700 further includes a data storage device 706 such as a magnetic disk or optical disk, coupled to bus 708 for storing information and instructions. Computer system 700 may be coupled via input/output module 710 to various devices (e.g., touchscreen 214). The input/output module 710 can be any input/output module. Example input/output modules 710 include data ports such as USB ports. In addition, input/output module 710 may be provided in communication with processor 702, so as to enable near area communication of computer system 700 with other devices. The input/output module 710 may provide, for example, wired communication in some implementations, or wireless communication in other implementations, and multiple interfaces may also be used. The input/output module 710 is configured to connect to a communications module 712. Example communications modules 712 (e.g., network module 216) include networking interface cards, such as Ethernet cards and modems.

The components of the system can be interconnected by any form or medium of digital data communication (e.g., a communication network). The communication network (e.g., communication network 150) can include, for example, any one or more of a personal area network (PAN), a local area network (LAN), a campus area network (CAN), a metropolitan area network (MAN), a wide area network (WAN), a broadband network (BBN), the Internet, and the like. Further, the communication network can include, but is not limited to, for example, any one or more of the following network topologies, including a bus network, a star network, a ring network, a mesh network, a star-bus network, tree or hierarchical network, or the like. The communications modules can be, for example, modems or Ethernet cards.

For example, in certain aspects, communications module 712 can provide a two-way data communication coupling to a network link that is connected to a local network. Wireless links and wireless communication may also be implemented. Wireless communication may be provided under various modes or protocols, such as GSM (Global System for Mobile Communications), Short Message Service (SMS), Enhanced Messaging Service (EMS), or Multimedia Messaging Service (MMS) messaging, CDMA (Code Division Multiple Access), Time division multiple access (TDMA), Personal Digital Cellular (PDC), Wideband CDMA, General Packet Radio Service (GPRS), or LTE (Long-Term Evolution), among others. Such communication may occur, for example, through a radio-frequency transceiver. In addition, short-range communication may occur, such as using a BLUETOOTH, WI-FI, or other such transceiver.

In any such implementation, communications module 712 sends and receives electrical, electromagnetic, or optical signals that carry digital data streams representing various types of information. The network link typically provides data communication through one or more networks to other data devices. For example, the network link of the communications module 712 may provide a connection through local network to a host computer or to data equipment operated by an Internet Service Provider (ISP). The ISP in turn provides data communication services through the world wide packet data communication network now commonly referred to as the "Internet." The local network and Internet both use electrical, electromagnetic, or optical signals that carry digital data streams. The signals through the various networks and the signals on the network link and through communications module 712, which carry the digital data to and from computer system 700, are example forms of transmission media.

Computer system 700 can send messages and receive data, including program code, through the network(s), the network link, and communications module 712. In the Internet example, a server might transmit a requested code for an application program through the Internet, the ISP, the local network, and communications module 712. The received code may be executed by processor 702 as it is received, and/or stored in data storage 706 for later execution.

In certain aspects, the input/output module 710 is configured to connect to a plurality of devices, such as an input device 714 (e.g., touchscreen 214) and/or an output device 716 (e.g., touchscreen 214). Example input devices 714 include a keyboard and a pointing device, e.g., a mouse or a trackball, by which a user can provide input to the computer system 700. Other kinds of input devices 714 can be used to provide for interaction with a user as well, such as a tactile input device, visual input device, audio input device, or brain-computer interface device. For example, feedback provided to the user can be any form of sensory feedback, e.g., visual feedback, auditory feedback, or tactile feedback, and input from the user can be received in any form, including acoustic, speech, tactile, or brain wave input. Example output devices 716 include display devices, such as an LED (light emitting diode), CRT (cathode ray tube), LCD (liquid crystal display) screen, a TFT LCD (Thin-Film-Transistor Liquid Crystal Display) or an OLED (Organic Light Emitting Diode) display, for displaying information to the user. The output device 716 may include appropriate circuitry for driving the output device 716 to present graphical and other information to a user.

According to one aspect of the present disclosure, the client 110A can be implemented using a computer system 700 in response to processor 702 executing one or more sequences of one or more instructions contained in memory 704. Such instructions may be read into memory 704 from another machine-readable medium, such as data storage device 706. Execution of the sequences of instructions contained in main memory 704 causes processor 702 to perform the process steps described herein. One or more processors in a multi-processing arrangement may also be employed to execute the sequences of instructions contained in memory 704. Processor 702 may process the executable instructions and/or data structures by remotely accessing the computer program product, for example by downloading the executable instructions and/or data structures from a remote server through communications module 712 (e.g., as in a cloud-computing environment). In alternative aspects, hard-wired circuitry may be used in place of or in combination with software instructions to implement various aspects of the present disclosure. Thus, aspects of the present disclosure are not limited to any specific combination of hardware circuitry and software.

Various aspects of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. For example, some aspects of the subject matter described in this specification may be performed on a cloud-computing environment. Accordingly, in certain aspects, a user of systems and methods as disclosed herein may perform at least some of the steps by accessing a cloud server through a network connection. Further, data files, circuit diagrams, performance specifications, and the like resulting from the disclosure may be stored in a database server in the cloud-computing environment, or may be downloaded to a private storage device from the cloud-computing environment.

Computing system 700 can include clients and servers. A client and server are generally remote from each other and typically interact through a communication network. The relationship of client and server arises by virtue of computer programs running on the respective computers and having a client-server relationship to each other. Computer system 700 can be, for example, and without limitation, a desktop computer, laptop computer, or tablet computer. Computer system 700 can also be embedded in another device, for example, and without limitation, a mobile telephone, a personal digital assistant (PDA), a mobile audio player, a Global Positioning System (GPS) receiver, a video game console, and/or a television set top box.

The term "machine-readable storage medium" or "computer-readable medium" as used herein refers to any medium or media that participates in providing instructions or data to processor 702 for execution. The term "storage medium" as used herein refers to any non-transitory media that store data and/or instructions that cause a machine to operate in a specific fashion. Such a medium may take many forms, including, but not limited to, non-volatile media, volatile media, and transmission media. Non-volatile media include, for example, optical disks, magnetic disks, or flash memory, such as data storage device 706. Volatile media include dynamic memory, such as memory 704. Transmission media include coaxial cables, copper wire, and fiber optics, including the wires that include bus 708. Common forms of machine-readable media include, for example, a floppy disk, a flexible disk, a hard disk, magnetic tape, any other magnetic medium, a CD-ROM, a DVD, any other optical medium, punch cards, paper tape, any other physical medium with patterns of holes, a RAM, a PROM, an EPROM, a FLASH EPROM, any other memory chip or cartridge, or any other medium from which a computer can read. The machine-readable storage medium can be a machine-readable storage device, a machine-readable storage substrate, a memory device, a composition of matter effecting a machine-readable propagated signal, or a combination of one or more of them.

As used in this specification of this application, the terms "computer-readable storage medium" and "computer-readable media" are entirely restricted to tangible, physical objects that store information in a form that is readable by a computer. These terms exclude any wireless signals, wired download signals, and any other ephemeral signals. Storage media is distinct from but may be used in conjunction with transmission media. Transmission media participates in transferring information between storage media. For example, transmission media includes coaxial cables, copper wire, and fiber optics, including the wires that include bus 708. Transmission media can also take the form of acoustic or light waves, such as those generated during radio-wave and infra-red data communications. Furthermore, as used in this specification of this application, the terms "computer," "server," "processor," and "memory" all refer to electronic or other technological devices. These terms exclude people or groups of people. For the purposes of the specification, the terms display or displaying means displaying on an electronic device.

In one aspect, a method may be an operation, an instruction, or a function and vice versa. In one aspect, a clause or a claim may be amended to include some or all of the words (e.g., instructions, operations, functions, or components) recited in other one or more clauses, one or more words, one or more sentences, one or more phrases, one or more paragraphs, and/or one or more claims.

To illustrate the interchangeability of hardware and software, items such as the various illustrative blocks, modules, components, methods, operations, instructions, and algorithms have been described generally in terms of their functionality. Whether such functionality is implemented as hardware, software, or a combination of hardware and software depends upon the particular application and design constraints imposed on the overall system. Skilled artisans may implement the described functionality in varying ways for each particular application.

The word "exemplary" is used herein to mean "serving as an example, instance, or illustration." Any embodiment described herein as "exemplary" is not necessarily to be construed as preferred or advantageous over other embodiments. Phrases such as an aspect, the aspect, another aspect, some aspects, one or more aspects, an implementation, the implementation, another implementation, some implementations, one or more implementations, an embodiment, the embodiment, another embodiment, some embodiments, one or more embodiments, a configuration, the configuration, another configuration, some configurations, one or more configurations, the subject technology, the disclosure, the present disclosure, other variations thereof and alike are for convenience and do not imply that a disclosure relating to such phrase(s) is essential to the subject technology or that such disclosure applies to all configurations of the subject technology. A disclosure relating to such phrase(s) may apply to all configurations, or one or more configurations. A disclosure relating to such phrase(s) may provide one or more examples. A phrase such as an aspect or some aspects may refer to one or more aspects and vice versa, and this applies similarly to other foregoing phrases.

A reference to an element in the singular is not intended to mean "one and only one" unless specifically stated, but rather "one or more." Pronouns in the masculine (e.g., his) include the feminine and neuter gender (e.g., her and its) and vice versa. The term "some" refers to one or more. Underlined and/or italicized headings and subheadings are used for convenience only, do not limit the subject technology, and are not referred to in connection with the interpretation of the description of the subject technology. Relational terms such as first, second, and the like may be used to distinguish one entity or action from another without necessarily requiring or implying any actual such relationship or order between such entities or actions. All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public, regardless of whether such disclosure is explicitly recited in the above description. No claim element is to be construed under the provisions of 35 U.S.C. § 112, sixth paragraph, unless the element is expressly recited using the phrase "means for" or, in the case of a method claim, the element is recited using the phrase "step for."

While this specification contains many specifics, these should not be construed as limitations on the scope of what may be claimed, but rather as descriptions of particular implementations of the subject matter. Certain features that are described in this specification in the context of separate embodiments can also be implemented in combination in a single embodiment. Conversely, various features that are described in the context of a single embodiment can also be implemented in multiple embodiments separately, or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

The subject matter of this specification has been described in terms of particular aspects, but other aspects can be implemented and are within the scope of the following claims. For example, while operations are depicted in the drawings in a particular order, this should not be understood as requiring that such operations be performed in the particular order shown or in sequential order, or that all illustrated operations be performed, to achieve desirable results. The actions recited in the claims can be performed in a different order and still achieve desirable results. As one example, the processes depicted in the accompanying figures do not necessarily require the particular order shown, or sequential order, to achieve desirable results. In certain circumstances, multitasking and parallel processing may be advantageous. Moreover, the separation of various system components in the aspects described above should not be understood as requiring such separation in all aspects, and it should be understood that the described program components and systems can generally be integrated together in a single software product or packaged into multiple software products.

The title, background, brief description of the drawings, abstract, and drawings are hereby incorporated into the disclosure and are provided as illustrative examples of the disclosure, not as restrictive descriptions. It is submitted with the understanding that they will not be used to limit the scope or meaning of the claims. In addition, in the detailed description, it can be seen that the description provides illustrative examples and the various features are grouped together in various implementations for the purpose of streamlining the disclosure. The method of disclosure is not to be interpreted as reflecting an intention that the claimed subject matter requires more features than are expressly recited in each claim. Rather, as the claims reflect, inventive subject matter lies in less than all features of a single disclosed configuration or operation. The claims are hereby incorporated into the detailed description, with each claim standing on its own as a separately claimed subject matter.

The claims are not intended to be limited to the aspects described herein, but are to be accorded the full scope consistent with the language claims and to encompass all legal equivalents. Notwithstanding, none of the claims are intended to embrace subject matter that fails to satisfy the requirements of the applicable patent law, nor should they be interpreted in such a way.

## Claims

1. A computer-implemented method for providing a touchscreen-based hand dexterity test, the method comprising:

   providing for display, on a touchscreen, a model path;
   receiving, from the touchscreen, a plurality of user touch points;
   fitting the plurality of user touch points to a user path;
   sampling an array of user points along the user path and sampling an array of model points along the model path;
   associating model points in the array of model points with corresponding user points in the array of user points, wherein:

   a next model point is associated with a next user point based on a present user point being at least a user interval distance from the next user point on the user path;
   the user interval distance comprises a model interval distance from a present model point to the next model point on the model path; and
   the user interval distance comprises a correction component based on an interval error that is a scalar product of a first vector, from the present model point to the present user point, and a second vector, from the present model point to the next model point; and

   determining a test result based on the associating.

2. The method of Claim 1, wherein the determining comprises:

   storing, in a single dimensional array, a magnitude of the first vector from each model point of the array of model points; and
   analyzing the single dimensional array to determine the test result.

3. The method of Claim 2, wherein the analyzing uses a global distance comprising a sum of the single dimensional array.

4. The method of Claim 1, wherein the model path comprises a spiral shape.

5. The method of Claim 1, wherein the correction component is applied over a plurality of model points.

6. The method of Claim 1, wherein adjacent points in the array of model points are separated by a constant interval distance.

7. The method of Claim 1, wherein the model path and the user path each comprise composite Bezier curves.

8. A system for providing a touchscreen-based hand dexterity test, the system comprising:

a touchscreen;
a memory comprising a model path; and
a processor configured to execute instructions which, when executed, cause the processor to:

provide for display, on the touchscreen, the model path;
receive, from the touchscreen, a plurality of user touch points;
fit the plurality of user touch points to a user path;
sample an array of user points along the user path and sample an array of model points along the model path;
associate model points in the array of model points with corresponding user points in the array of user points, wherein:

a next model point is associated with a next user point based on a present user point being at least a user interval distance from the next user point on the user path;
the user interval distance comprises a model interval distance from a present model point to the next model point on the model path; and
the user interval distance comprises a correction component based on an interval error that is a scalar product of a first vector, from the present model point to the present user point, and a second vector, from the present model point to the next model point;

store, in the memory, a distance array comprising the first vector from each model point of the array of model points; and
analyze the distance array to determine a test result.

9. The system of Claim 8, wherein the distance array is a single dimensional array comprising a magnitude of the first vector from each model point of the array of model points.

10. The system of Claim 9, wherein the processor is configured to analyze the distance array by using a global distance comprising a sum of the distance array to determine the test result.

11. The system of Claim 8, wherein the model path comprises a spiral shape.

12. The system of Claim 8, wherein the processor is configured to apply the correction component over a plurality of model points.

13. The system of Claim 8, wherein adjacent points in the array of model points are separated by a constant interval distance.

14. The system of Claim 8, wherein the model path and the user path each comprise composite Bezier curves.

15. A non-transitory machine-readable storage medium comprising machine-readable instructions for causing a processor to execute a method for providing a touchscreen-based hand dexterity test, comprising:

providing for display, on a touchscreen, a model path;
receiving, from the touchscreen, a plurality of user touch points;
fitting the plurality of user touch points to a user path;
sampling an array of user points along the user path and sampling an array of model points along the model path;
associating model points in the array of model points with corresponding user points in the array of user points, wherein:

a next model point is associated with a next user point based on a present user point being at least a user interval distance from the next user point on the user path;
the user interval distance comprises a model interval distance from a present model point to the next model point on the model path; and
the user interval distance comprises a correction component based on an interval error that is a scalar product of a first vector, from the present model point to the present user point, and a second vector, from the present model point to the next model point, wherein the correction component is applied over a plurality of model points; and

determining a test result based on the associating.

FIG. 1

FIG. 2

300

START

311

PROVIDE FOR DISPLAY, ON A TOUCHSCREEN, A MODEL PATH

312

RECEIVE, FROM THE TOUCHSCREEN, A PLURALITY OF USER TOUCH POINTS

313

FIT THE PLURALITY OF USER TOUCH POINTS TO A USER PATH

314

SAMPLE AN ARRAY OF USER POINTS ALONG THE USER PATH AND SAMPLE AN ARRAY OF MODEL POINTS ALONG THE MODEL PATH

315

ASSOCIATE MODEL POINTS IN THE ARRAY OF MODEL POINTS WITH CORRESPONDING USER POINTS IN THE ARRAY OF USER POINTS, WHEREIN: A NEXT MODEL POINT IS ASSOCIATED WITH A NEXT USER POINT BASED ON A PRESENT USER POINT BEING AT LEAST A USER INTERVAL DISTANCE FROM THE NEXT USER POINT ON THE USER PATH; WHEREIN THE USER INTERVAL DISTANCE INCLUDES A MODEL INTERVAL DISTANCE FROM A PRESENT MODEL POINT TO THE NEXT MODEL POINT ON THE MODEL PATH; AND WHEREIN THE USER INTERVAL DISTANCE INCLUDES A CORRECTION COMPONENT BASED ON AN INTERVAL ERROR THAT IS A SCALAR PRODUCT OF A FIRST VECTOR, FROM THE PRESENT MODEL POINT TO THE PRESENT USER POINT, AND A SECOND VECTOR, FROM THE PRESENT MODEL POINT TO THE NEXT MODEL POINT

316

DETERMINE A TEST RESULT BASED ON THE ASSOCIATING

END

FIG. 3A

320

START

331

RETRIEVE A PRESENT MODEL POINT FROM THE ARRAY OF MODEL POINTS

332

DETERMINE A MODEL INTERVAL DISTANCE FROM THE PRESENT MODEL POINT TO A NEXT MODEL POINT ON THE MODEL PATH

333

DETERMINE AN INTERVAL ERROR FOR THE PRESENT USER POINT THAT IS A SCALAR PRODUCT OF A FIRST VECTOR FROM THE PRESENT MODEL POINT TO A PRESENT USER POINT AND A SECOND VECTOR FROM THE PRESENT MODEL POINT TO THE NEXT MODEL POINT

334

DETERMINE A CORRECTION FACTOR BASED ON THE INTERVAL ERROR

335

DETERMINE A USER INTERVAL DISTANCE AS INCLUDING THE MODEL INTERVAL DISTANCE AND THE CORRECTION FACTOR

336

ASSOCIATE THE NEXT MODEL POINT WITH A NEXT USER POINT BASED ON THE PRESENT USER POINT BEING AT LEAST THE USER INTERVAL DISTANCE FROM THE NEXT USER POINT ON THE USER PATH

337

YES — UNREAD POINTS IN THE ARRAY OF MODEL POINTS?

NO

END

FIG. 3B

214A

Instructions to patient:
Please trace the path with your finger in the indicated direction.

228A

460

# FIG. 4A

214A

Calculating dexterity test score...

Your average distance from the model path is 2 cm
Your dexterity test result is: Excellent
Sending results to your care provider...

228

242

FIG. 4B

FIG. 5A

FIG. 5B

FIG. 5C

FIG. 6

700

| PROCESSOR 702 | MEMORY 704 | DATA STORAGE 706 |

BUS 708

| INPUT/OUTPUT MODULE 710 |

| COMMUNICATIONS MODULE 712 | INPUT DEVICE 714 | OUTPUT DEVICE 716 |

## FIG. 7

Europäisches
Patentamt

European
Patent Office

Office européen
des brevets

# EUROPEAN SEARCH REPORT

Application Number

EP 18 29 0007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/263146 A1 (AGGARWAL VARUN [IN] ET AL) 14 September 2017 (2017-09-14) * paragraph [0006] - paragraph [0016] * * paragraph [0023] - paragraph [0030] * * figures 1,5,7,8 * ----- | 1-15 | INV. G16H50/30 G16H50/20 |
| X | US 2012/330182 A1 (ALBERTS JAY L [US] ET AL) 27 December 2012 (2012-12-27) * paragraph [0009] - paragraph [0027] * * paragraph [0086] - paragraph [0091] * * paragraph [0123] - paragraph [0145] * * figures 8-20 * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 28 May 2018 | Wittke, Claudia |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 18 29 0007

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2018

| Patent document cited in search report | Publication date | Patent family member(s) | Publication date |
|---|---|---|---|
| US 2017263146 A1 | 14-09-2017 | NONE | |
| US 2012330182 A1 | 27-12-2012 | US 2012330182 A1<br>WO 2011063248 A1 | 27-12-2012<br>26-05-2011 |

EPO FORM P0459

For more details about this annex : see Official Journal of the European Patent Office, No. 12/82